# EUROPÄISCHE PATENTSCHRIFT

(11) **EP 0 653 932 B1**
(45) Veröffentlichungstag und Bekanntmachung des Hinweises auf die Patenterteilung: **19.06.1996**
(21) Anmeldenummer: 93917653.3
(22) Anmeldetag: 26.07.1993
(51) Int. Cl.: A61K 7/42

(54) **HAUTBRÄUNUNGSMITTEL**
ARTIFICIAL-TAN AGENTS FOR THE SKIN
AGENT BRONZANT POUR LA PEAU

(30) Priorität: 05.08.1992 DE 4225908
(43) Veröffentlichungstag der Anmeldung: 24.05.1995
(73) Patentinhaber: Henkel Kommanditgesellschaft auf Aktien, 40191 Düsseldorf (DE)
(72) Erfinder: SCHUMANN, Klaus, D-40699 Erkrath (DE); HEIDE-KOSSMANN, Barbara, D-41239 Mönchengladbach (DE); KNÜBEL, Georg, D-40229 Düsseldorf (DE); BUSCH, Peter, D-40699 Erkrath (DE)
(86) Internationale Anmeldenummer: EP9301985
(87) Internationale Veröffentlichungsnummer: WO9403148

(56) Entgegenhaltungen:
- EP-A- 0 207 287
- EP-A- 0 425 324
- WO-A-91/17739

## Beschreibung

Gegenstand der Erfindung ist die kosmetische Verwendung von Indolinderivaten, insbesondere 5,6-Dihydroxyindolin, als Hautbräunungsmittel sowie Hautbräunungsmittel-Zubereitungen enthaltend Indolinderivate und Dihydroxyaceton.

Trotz des hautschädigenden Einflusses ultravioletter Strahlung ist eine gebräunte Haut immer noch ein in westlichen Ländern verbreitetes Schönheitsideal. Es hat daher nicht an Versuchen gefehlt, durch die Applikation von Hautbräunungspräparaten eine möglichst natürlich aussehende braune Färbung der Haut auch ohne Einwirkung von ultravioletter Strahlung zu erzielen.

Man kann 3 verschiedene Typen von Hautbräunungspräparaten unterscheiden:
1. Hautbräunungspräparate auf Basis von Pflanzenextrakten
   Die hautbräunenden Bestandteile in diesen Mitteln sind Naphthochinonderivate, z. B. Lawson (aus Henna) oder Juglon (aus der Walnuß) und haften durch nicht-kovalente Wechselwirkungen auf der äußeren Hautschicht. Die Stabilität der so erzielten Färbungen ist deshalb gering; so ist beispielsweise die Schweißbeständigkeit nicht zufriedenstellend.
2. Hautbräunungspräparate auf Basis von Dihydroxyaceton
   Dihydroxyaceton reagiert als reduzierender Zucker mit den Aminosäuren der Haut bzw. den freien Amino- und Iminogruppen des Keratins über eine Reihe von Zwischenstufen im Sinne einer Maillard-Reaktion zu braungefärbten Stoffen, sogenannten Melanoiden. Diese Bräune ist nicht abwaschbar und wird erst mit der normalen Abschuppung der Haut entfernt. Ein Nachteil dieser Methode ist der gelbbraune Farbton, der unnatürlich wirkt und daher ästhetisch nicht befriedigend ist. Gemeinsamer Nachteil von Pflanzenextrakten und Dihydroxyaceton enthaltenden Hautbräunungsmitteln ist die im Gegensatz zur natürlichen Pigmentierung mangelnde Lichtschutzwirkung der Färbungen.
3. Hautbräunungspräparate auf Basis von Melanin-Vorläufern
   Die US-Patentschriften 4 515 773, 5 061 480, 4 021 538 und die europäischen Offenlegungsschriften EP-318 369 und EP-328 099 beschreiben die Anwendung biosynthetisch "entfernter" Vorstufen des Melanins, wie z. B. Phenylalanin, Tyrosin oder Dopa. Diese haben jedoch nur schwache färberische Eigenschaften. Der Zusatz von die Melaninbiosynthese katalysierenden Enzymen, z. B. Tyrosinase, ist aus toxikologischen Gründen bedenklich. In den Patentschriften US 5 037 640, US 5 049 381 und EP 239 826 sowie in der europäischen Offenlegungsschrift EP-356 119 wird der Schlüsselbaustein der Melaninbiosynthese 5,6-Dihydroxyindol als Farbstoffvorläufer vorgeschlagen. Diese Verbindung bereitet jedoch wegen ihrer hohen Oxidationsempfindlichkeit große anwendungstechnische Probleme.

Aus der deutschen Offenlegungsschrift DE 40 16 177 ist bekannt, Indolinderivate als Haarfarbstoffvorprodukte zu verwenden.

Aufgabe der vorliegenden Erfindung ist es, eine Hautbräunungsmittel-Zubereitung auf der Basis von lagerstabilen, gut handhabbaren "nahen Vorstufen" des Melanins zu konzipieren.

Unter Hautbräunungsmittel-Zubereitungen im Sinne der Erfindung sind topisch anzuwendende Mittel zu verstehen, die der Haut ohne zusätzliche Einwirkung von UV-Strahlen eine der natürlichen Sonnenbräunung gleich- oder nahekommende Bräunung verleihen.

Es wurde nun eine neue und überraschende Möglichkeit gefunden, Hautbräunungen unter Verwendung von Indolinderivaten der Formel I zu erzielen. Gegenstand der Erfindung ist die Verwendung von Indolinen der Formel I 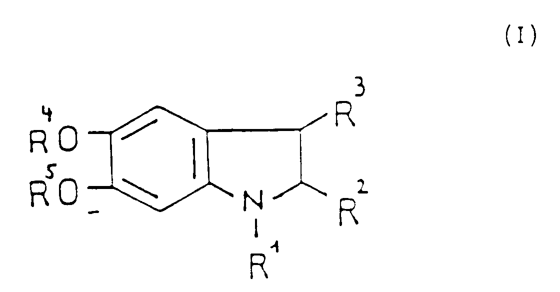
worin R¹, R², R³, R⁴ und R⁵ unabhängig voneinander Wasserstoff oder Alkylgruppen mit 1 bis 4 C-Atomen oder R⁴ und R⁵ Hydroxyalkylgruppen mit 2 bis 4 C-Atomen oder gemeinsam mit den Sauerstoffatomen, an die sie gebunden sind, eine Alkylendioxygruppe mit 1 bis 4 C-Atomen darstellen oder deren Salzen zur Hautbräunung.

Bevorzugt geeignet sind monomethylsubstituierte 5,6-Dihydroxyindolinderivate, also jene Indoline der Formel I, in welchen eine der Gruppen R¹, R² und R³ eine Methylgruppe ist und die übrigen Wasserstoff sind. Vor allem aber eignet sich der Grundkörper 5,6-Dihydroxyindolin selbst.
5,6-Dihydroxyindolin wird an der Luft schnell zu 5,6-Dihydroxyindol und anschließend zu melaninähnlichen Farbstoffen oxidiert.

In Form ihrer Salze können Indoline der Formel I auch in technischem Maßstab an der Luft gehandhabt werden. Geeignete Salze sind die Sulfate, Phosphate, Acetate, Propionate, Lactate, Citrate, Hydrochloride und vor allem die Hydrobromide.

Die Indoline der Formel I bilden auf der Haut nach einer Einwirkzeit von ca. 30 Minuten einen ansprechenden neutralbraunen Farbton mit guten Echtheitseigenschaften, wie Schweißechtheit, Waschechtheit und Lichtechtheit. Aufgrund der Bildung eines melaninähnlichen Farbstoffs ist eine Schutzfunktion gegen Ultraviolettstrahlung zu erwarten.
Der einfachste Fall einer aus einem Indolin der Formel I hergestellten Hautbräunungsmittel-Zubereitung ist eine wäßrige oder wäßrig/alkoholische Lösung eines Indolins der Formel I oder dessen Salz. Üblicherweise werden die Indoline der Formel I oder deren Salze jedoch in einen wäßrigen kosmetischen Träger eingearbeitet. Die Indoline der Formel I oder deren Salze werden dabei in Mengen von 0,01 bis 10 mMol, vorzugsweise 0,1 bis 3 mMol, pro 100 g der gesamten Hautbräunungsmittel-Zubereitung zugegeben.

Der pH-Wert der Zubereitungen kann im Bereich von 3 bis 11 liegen, vorzugsweise liegt er jedoch zwischen 4 und 7,5. Der schwach saure bis neutrale Bereich ist dem alkalischen Bereich zum einen wegen der besseren Hautverträglichkeit, zum anderen wegen der in diesem Bereich höheren Stabilität der Indoline der Formel I gegen Luftsauerstoffoxidation vorzuziehen.

Ein weiterer Patentgegenstand ist daher die Verwendung von Indolinen der Formel I oder deren Salze zur Hautbräunung, wobei die Indoline in Form einer wäßrigen kosmetischen Zubereitung mit einem pH-Wert von 4,0 bis 7,5 und einem Gehalt an Indolin von 0,01 bis 10 mMol, vorzugsweise 0,1 bis 3 mMol, pro 100 g der Zubereitung auf die Haut aufgebracht werden.

Die Indoline der Formel I können auch als eine Mischung von verschiedenen Indolinen der Formel I zum Einsatz kommen.

Wäßrige, kosmetische Träger können Wasser-in-Öl- oder Öl- in-Wasser-Emulsionen oder auch Gele sein. Wasser-in-Öl-Emulsionen als Träger sind bevorzugt bei Personen mit trockener oder reifer Haut; sie enthalten Öl- bzw. Fettkomponenten in einer Menge von 5 - 70 Gew.% bezogen auf die gesamte Hautbräunungsmittel-Zubereitung. Öl-in-Wasser-Emulsionen als Träger verwendet man vorzugsweise bei jugendlicher oder normaler bis fetter Haut; sie enthalten Öl- bzw. Fettkomponenten in einer Menge von 1 - 35 Gew.% bezogen auf die gesamte Hautbräunungsmittel-Zubereitung. Wäßrige Gele erlauben die fettarme Applikation der Indolin-Hautbräunungsmittel.

Ein weiterer Patentgegenstand ist die Verwendung von Indolinen der Formel I oder deren Salzen zur Hautbräunung, wobei die wäßrige kosmetische Zubereitung eine Öl-in-Wasser-Emulsion oder ein Gel ist.

Als Öl- oder Fettkomponenten kommen alle für kosmetische Zubereitungen üblichen Öle, Fette oder auch Wachse in Frage, z. B. Fettsäureester wie Isopropylisostearat, Isopropylpalmitat, Isopropylmyristat, Myristylmyristat, langkettige Alkohole wie 2-Octyldodecanol, Cetyl- und Stearylalkohol, ferner Triglyceride, Wachse wie z. B. Bienenwachs oder Wollwachs, mineralische Öle wie z. B. Paraffinöl, Vaseline, pflanzliche Öle wie z. B. Weizenkeimöl, Erdnußöl Rizinusöl, Mandelöl, Avocadoöl, Jojobaöl, aber auch Siliconöle. Als Emulgatoren kommen anionische Emulgatoren, wie z. B. Seifen, insbesondere die Alkali- oder Alkanolaminseifen von linearen C₁₂₋₁₈-Fettsäuren, Fettalkoholsulfate und nichtionische Emulgatoren, insbesondere Anlagerungsprodukte von 3 bis 50 Mol Ethylenoxid an Fettalkohole, an Alkylphenole, an Fettsäuren, an Fettsäurepartialglyceride, an Fettsäuresorbitanpartialester, an Fettsäuremethylglucosidpartialester, ferner Alkylglucoside, Fettsäurepolyglycerinester und Polyethylenglycol/Langkettendiol-Polymere.
Weitere Konfektionierungsmittel können sein: Verdickungsmittel, z. B. wasserlösliche Polymere wie z. B. Celluloseether, Stärke und Stärkeether, Pflanzengumme, Guar-Gum, Alginate, Xanthan-Gum, synthetische wasserlösliche Polymere, wie z. B. Polyacrylsäure oder auch Metallseifen,
Antioxidantien, wie z. B. Ascorbinsäure, Natriumsulfit, Vitamin E, substituierte Phenole wie Butylmethoxyphenol,
Komplexbildner, z. B. 1-Hydroxyethan-1,1-diphosphonsäure, Nitrilotriessigsaure oder Ethylendiamintetraessigsäure oder deren Salze, Konservierungsmittel, z. B. p-Hydroxybenzoesäureester,
pH-Stellmittel, wie z. B. Citronensäure oder Milchsäure, Parfumöle, hautkosmetische Hilfsmittel wie Hautfeuchthaltemittel, z. B. Pyrrolidoncarbonsäure und Milchsäure sowie deren Salze, Glycerin, Polyethylenglycol, 1,2-Propylenglycol, Diethylenglycol, Sorbit, Harnstoff, Polysaccharide, Kollagen und Hyaluronsäure, weiterhin rückfettende Mittel, z. B. Ethylenoxidanlagerungsprodukte von Fettsäuren oder Fettsäureglycerinestern,
ferner Proteinderivate, D-Panthenol, Cholesterin, Vitamine, Ceramide oder Pflanzenextrakte.

Zur Beschleunigung der Bildung melaninähnlicher Farbstoffe können den Hautbräunungsmittel-Zubereitungen auch Metallsalze als Oxidationskatalysatoren zugegeben werden. Ein weiterer Erfindungsgegenstand ist die Verwendung von Indolinen der Formel I oder deren Salzen zur Hautbräunung, wobei die Indoline in Form einer wäßrigen kosmetischen Zubereitung, enthaltend ein Hauptgruppen-, Übergangs- oder Lanthanidenmetallsalz ausgewählt aus der Gruppe Lithium, Natrium, Kalium, Magnesium, Calcium, Strontium, Aluminium, Titan, Vanadium, Mangan, Eisen, Kobalt, Kupfer, Silber, Gold, Zink, Cer, Lanthan, Europium, Gadolinium und Dysprosium, auf die Haut aufgebracht werden. Mogliche Anionen sind z. B. Chloride, Bromide, Sulfate, Oxide, Acetate, Lactate, Citrate, Propionate, Nitrate und Nitrite.

Besonders bevorzugt sind jedoch toxikologisch unbedenkliche Metallsalze ohne oxidierend wirkendes Anion. Ein weiterer Erfindungsgegenstand ist deshalb die Verwendung von Indolinen in Form einer wäßrigen kosmetischen Zubereitung, die ein Chlorid, Bromid, Sulfat, Acetat, Lactat oder Citrat des Lithiums, Magnesiums, Calciums, Aluminiums, Eisens, Cers oder Lanthans enthält.

Es ist zu beachten, daß Magnesiumsulfat und Natriumchlorid gleichzeitig als Emulsionsstabilisatoren für Wasser-in-Öl-Emulsionen verwendet werden.
Im gegebenen Falle sind die Metallsalze in einer Menge von 0,1 bis 50 mMol, vorzugsweise 0,3 bis 12,5 mMol, pro 100 g der gesamten Hautbräunungsmittel-Zubereitung enthalten.

Zur Erzielung von speziellen Brauntönen können neben den Indolinen der Formel I auch andere, übliche hautbräunende Substanzen verwendet werden. Solche Substanzen sind z. B. Pflanzenextrakte der Walnuß, des Hennastrauches und der Ratanhiawurzel, vorzugsweise jedoch werden die Indoline der Formel I gemeinsam mit Hydroxymethylketonen und -aldehyden, wie z. B. Dihydroxyaceton, Erythrulose oder Glycerinaldehyd verwendet.

Ganz besonders bevorzugt ist Dihydroxyaceton. Ein weiterer Patentgegenstand sind deshalb Hautbräunungsmittel-Zubereitungen enthaltend Indoline der Formel I oder deren Salze in einer Menge von 0,01 bis 10 mMol, vorzugsweise 0,1 bis 3 mMol, und Dihydroxyaceton in einer Menge von 0,05 bis 10 g, vorzugsweise 0,2 bis 5 g, jeweils pro 100 g der Zubereitung, und einen wäßrigen kosmetischen Träger.

Die Indoline der Formel I enthaltenden Zubereitungen werden auf die Haut aufgebracht und durch Verreiben auf der Hautoberfläche verteilt. Dabei verwendet man für eine Gesichtsoberfläche ca. 3 g Hautbräunungsmittel. Nach einer Einwirkungszeit von 30 Minuten tritt eine sichtbare Bräunung ein, die bis zur natürlichen Abschuppung der Haut anhält.

Die folgenden Beispiele sollen den Patentgegenstand näher erläutern.

### Beispiele

**Tabelle 1**

| Wasser-in-Öl-Emulsion (pH = 4,6) | | |
|---|---|---|
| | 1a | 1b |
| Zinkstearat | 2,0 g | 2,0 g |
| Aluminiumstearat | 0,5 g | 0,5 g |
| Isopropylisostearat | 17,0 g | 17,0 g |
| Weizenkeimöl | 3,0 g | 3,0 g |
| Methylglucosedioleat | 2,0 g | 2,0 g |
| Polyethylenglykol-45/Dodecylglycol-Copolymer | 1,0 g | 1,0 g |
| Bienenwachs | 3,0 g | 3,0 g |
| 1,2-Dodecandiol | 1,0 g | 1,0 g |
| 4-Hydroxybenzoesaurepropylester | 0,2 g | 0,2 g |
| Milchsäure | 0,5 g | 0,5 g |
| Natriumhydroxid | 0,15 g | 0,15 g |
| 4-Hydroxybenzoesäuremethylester | 0,2 g | 0,2 g |
| Magnesiumsulfat x 7 H₂O | 0,7 g | 0,7 g |
| Glycerin | 2,0 g | 2,0 g |
| 5,6-Dihydroxyindolin-Hydrobromid | 0,9 mMol | 0,9 mMol |
| Dihydroxyaceton | - | 2,0 g |
| Wasser | ad 100 g | ad 100 g |

**Tabelle 2**

| Wasser-in-Öl-Emulsion (pH = 7) | | |
|---|---|---|
| | 2a | 2b |
| Zinkstearat | 2,0 g | 2,0 g |
| Aluminiumstearat | 0,5 g | 0,5 g |
| Isopropylisostearat | 17,0 g | 17,0 g |
| Weizenkeimöl | 3,0 g | 3,0 g |
| Methylglucosedioleat | 2,0 g | 2,0 g |
| Polyethylenglykol-45/Dodecylglycol-Copolymer | 1,0 g | 1,0 g |
| Bienenwachs | 3,0 g | 3,0 g |
| 1,2-Dodecandiol | 1,0 g | 1,0 g |
| 4-Hydroxybenzoesäurepropylester | 0,2 g | 0,2 g |
| 4-Hydroxybenzoesäuremethylester | 0,2 g | 0,2 g |
| Magnesiumsulfat x 7 H₂O | 0,7 g | 0,7 g |
| Glycerin | 2,0 g | 2,0 g |
| 5,6-Dihydroxyindolin-Hydrobromid | 0,9 mMol | 0,9 mMol |
| Dihydroxyaceton | - | 2,0 g |
| Wasser | ad 100 g | ad 100 g |

**Tabelle 3**

| Wasser-in-Öl-Emulsion (pH = 9) | | |
|---|---|---|
| | 3a | 3b |
| Zinkstearat | 2,0 g | 2,0 g |
| Aluminiumstearat | 0,5 g | 0,5 g |
| Isopropylisostearat | 17,0 g | 17,0 g |
| Weizenkeimöl | 3,0 g | 3,0 g |
| Methylglucosedioleat | 2,0 g | 2,0 g |
| Polyethylenglykol-45/Dodecylglycol-Copolymer | 1,0 g | 1,0 g |
| Bienenwachs | 3,0 g | 3,0 g |
| 1,2-Dodecandiol | 1,0 g | 1,0 g |
| 4-Hydroxybenzoesäurepropylester | 0,2 g | 0,2 g |
| Natriumhydroxid | 0,01 g | 0,01 g |
| 4-Hydroxybenzoesäuremethylester | 0,2 g | 0,2 g |
| Magnesiumsulfat x 7 H₂O | 0,7 g | 0,7 g |
| Glycerin | 2,0 g | 2,0 g |
| 5,6-Dihydroxyindolin-Hydrobromid | 0,9 mMol | 0,9 mMol |
| Dihydroxyaceton | - | 2,0 g |
| Wasser | ad 100 g | ad 100 g |

**Tabelle 4**

| Öl-in-Wasser-Emulsion | | |
|---|---|---|
| | 4a | 4b |
| Mono-/Diglycerid der Palmitin- und Stearinsäure | 10,0 g | 10,0 g |
| Cetyl/Stearylalkohol | 2,0 g | 2,0 g |
| Cetyl/Stearylalkohol + 12 Mol EO | 1,5 g | 1,5 g |
| Cetyl/Stearylalkohol + 30 Mol EO | 1,5 g | 1,5 g |
| Myristylmyristat | 5,0 g | 5,0 g |
| 2-Octyldodecanol | 10,0 g | 10,0 g |
| Paraffinöl perliquidum | 5,0 g | 5,0 g |
| Glycerin | 5,0 g | 5,0 g |
| Polyethylenglykol 400 | 1,6 g | 1,6 g |
| 4-Hydroxybenzoesäuremethylester | 0,2 g | 0,2 g |
| 4-Hydroxybenzoesäurepropylester | 0,2 g | 0,2 g |
| 5,6-Dihydroxyindolin-Hydrobromid | 0,9 mMol | 0,9 mMol |
| Dihydroxyaceton | - | 2,0 g |
| Wasser | ad 100 g | ad 100 g |

**Tabelle 5**

| Gel | | |
|---|---|---|
| | 5a | 5b |
| Polyacrylsäure oder Methylhydroxypropylcellulose | 0,8 g | 0,8 g |
| Ethanol | 20,0 g | 20,0 g |
| Sorbit | 5,0 g | 5,0 g |
| hydriertes Ricinusöl + 40 Mol EO | 1,0 g | 1,0 g |
| Polyethylenglycol-7-Glycerinkokosfettsäureester | 2,5 g | 2,5 g |
| α-Ethylhexancarbonsäure + 13 Mol EO | 1,0 g | 1,0 g |
| 5,6-Dihydroxyindolin-Hydrobromid | 0,9 mMol | 0,9 mMol |
| Dihydroxyaceton | - | 2,0 g |
| Wasser | ad 100 g | ad 100 g |

Es zeigte sich, daß 5,6-Dihydroxyindolin-haltige Zubereitungen, insbesondere diejenigen mit Dihydroxyaceton-Zusatz, die Haut intensiv anfärben und einen natürlich wirkenden Braunton ergeben.

## Patentansprüche

1. Kosmetische Verwendung von Indolinen der Formel I 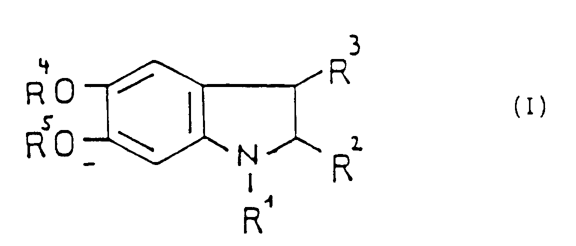 worin R¹, R², R³, R⁴ und R⁵ unabhängig voneinander Wasserstoff oder Alkylgruppen mit 1 bis 4 C-Atomen oder R⁴ und R⁵ Hydroxyalkylgruppen mit 2 bis 4 C-Atomen oder gemeinsam mit den Sauerstoffatomen, an die sie gebunden sind, eine Alkylendioxygruppe mit 1 bis 4 C-Atomen darstellen, oder deren Salzen zur Hautbräunung.

2. Verwendung nach Anspruch 1, dadurch gekennzeichnet, daß in der Formel I eine der Gruppen R¹, R² und R³ eine Methylgruppe ist und die übrigen Wasserstoff sind.

3. Verwendung nach Anspruch 1, dadurch gekennzeichnet, daß das Indolin der Formel I 5,6-Dihydroxyindolin ist.

4. Verwendung nach Anspruch 1 bis 3, dadurch gekennzeichnet, daß die Indoline der Formel I oder deren Salze in Form einer wäßrigen kosmetischen Zubereitung mit einem pH-Wert von 4,0 bis 7,5 und einem Gehalt an Indolin von 0,01 bis 10 mMol, vorzugsweise 0,1 bis 3 mMol, pro 100 g der Zubereitung auf die Haut aufgebracht werden.

5. Verwendung nach Anspruch 4, dadurch gekennzeichnet, daß die wäßrige kosmetische Zubereitung eine Öl-in-Wasser-Emulsion ist.

6. Verwendung nach Anspruch 4, dadurch gekennzeichnet, daß die wäßrige kosmetische Zubereitung ein Gel ist.

7. Verwendung nach Anspruch 1 bis 6, dadurch gekennzeichnet, daß die Indoline der Formel I oder deren Salze in Form einer wäßrigen kosmetischen Zubereitung, enthaltend ein Hauptgruppen-, Übergangs- oder Lanthaniden-Metallsalz ausgewählt aus der Gruppe Lithium, Natrium, Kalium, Magnesium, Calcium, Strontium, Aluminium, Titan, Vanadium, Mangan, Eisen, Kobalt, Kupfer, Silber, Gold, Zink, Cer, Lanthan, Europium, Gadolinium und Dysprosium, auf die Haut aufgebracht werden.

8. Verwendung nach Anspruch 7, dadurch gekennzeichnet, daß die wäßrige kosmetische Zubereitung ein Chlorid, Bromid, Sulfat, Acetat, Lactat oder Citrat des Lithiums, Magnesiums, Calciums, Aluminiums, Eisens, Cers oder Lanthans enthält.

9. Hautbräunungsmittel-Zubereitungen enthaltend Indoline der Formel I gemäß Anspruch 1 oder deren Salze in einer Menge von 0,01 bis 10 mMol, vorzugsweise 0,1 bis 3 mMol, und Dihydroxyaceton in einer Menge von 0,05 bis 10 g, vorzugsweise 0,2 bis 5 g, jeweils pro 100 g der Zubereitung, und einen wäßrigen kosmetischen Träger.

## Claims

1. The use of indolines corresponding to formula I: 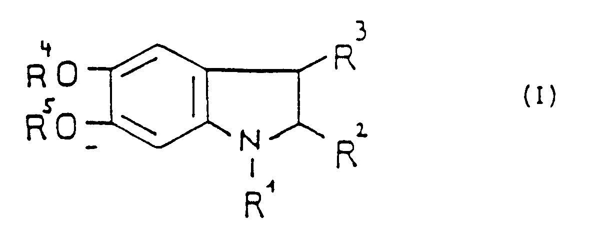 in which R¹, R², R³, R⁴ and R⁵ independently of one another represent hydrogen or alkyl groups containing 1 to 4 carbon atoms or R⁴ and R⁵ represent hydroxyalkyl groups containing 2 to 4 carbon atoms or, together with the oxygen atoms to which they are attached, represent an alkylene dioxy group containing 1 to 4 carbon atoms, or salts thereof for tanning the skin.

2. The use claimed in claim 1, characterized in that one of the groups R¹, R² and R³ in formula I is a methyl group and the others are hydrogen.

3. The use claimed in claim 1, characterized in that the indoline corresponding to formula I is 5,6-dihydroxyindoline.

4. The use claimed in claims 1 to 3, characterized in that the indolines corresponding to formula I or salts thereof are applied to the skin in the form of a water-based cosmetic preparation with a pH value of 4.0 to 7.5 and an indoline content of 0.01 to 10 mmoles and preferably 0.1 to 3 mmoles per 100 g of the preparation.

5. The use claimed in claim 4, characterized in that the water-based cosmetic preparation is an oil-in-water emulsion.

6. The use claimed in claim 4, characterized in that the water-based cosmetic preparation is a gel.

7. The use claimed in claims 1 to 6, characterized in that the indolines corresponding to formula I or salts thereof are applied to the skin in the form of a water-based cosmetic preparation containing a main group, transition or lanthanide metal salt selected from the group consisting of lithium, sodium, potassium, magnesium, calcium, strontium, aluminium, titanium, vanadium, manganese, iron, cobalt, copper, silver, gold, zinc, cerium, lanthanum, europium, gadolinium and dysprosium.

8. The use claimed in claim 7, characterized in that the water-based cosmetic preparation contains a chloride, bromide, sulfate, acetate, lactate or citrate, of lithium, magnesium, calcium, aluminium, iron, cerium or lanthanum.

9. Skin tanning preparations containing indolines corresponding to formula I or salts thereof in a quantity of 0.01 to 10 mmoles and preferably in a quantity of 0.1 to 3 mmoles, dihydroxyacetone in a quantity of 0.05 to 10 g and preferably in a quantity of 0.2 to 5 g per 100 g of the preparation and a water-based cosmetic carrier.

## Revendications

1. Utilisation cosmétique d'indolines de formule I : 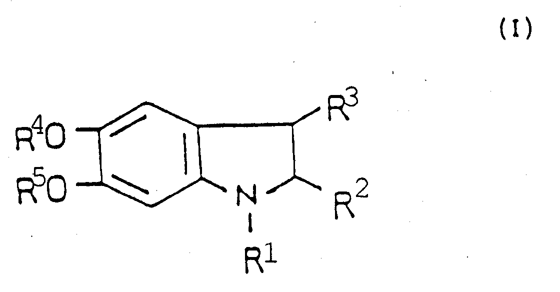 dans laquelle R¹, R², R³, R⁴ et R⁵ indépendamment l'un de l'autre, représentent de l'hydrogène ou des groupes alcoyle, ayant de 1 à 4 atomes de carbone ou bien R⁴ et R⁵ représentent des groupes hydroxyalcoyle ayant de 2 à 4 atomes de carbone, ou conjointement avec les atomes d'oxygène, auxquels ils sont liés, représentent un groupe alcoylènedioxy ayant de 1 à 4 atomes de carbone, ou de leurs sels, pour le bronzage de la peau.

2. Utilisation selon la revendication 1, caractérisée en ce que dans la formule I, R¹, R² et R³ sont un radical méthyle et les autres sont de l'hydrogène.

3. Utilisation selon la revendication 1, caractérisée en ce que l'indoline de formule I est la 5,6-dihydroxyindoline.

4. Utilisation selon les revendications 1 à 3, caractérisée en ce que l'indoline de formule I ou ses sels est appliquée sur la peau sous la forme d'une préparation cosmétique aqueuse ayant une valeur de pH allant de 4,0 à 7,5 et une teneur en indoline allant de 0,01 à 10 mmol, de préférence de 0,1 à 3 mmol, pour 100 g de préparation.

5. Utilisation selon la revendication 4, caractérisée en ce que la préparation cosmétique aqueuse est une émulsion d'huile dans l'eau.

6. Utilisation selon la revendication 4, caractérisée en ce que la préparation cosmétique est un gel.

7. Utilisation selon les revendications 1 à 6, caractérisée en ce que les indolines de formule I, ou leurs sels sont appliquées sur la peau sous la forme d'une préparation cosmétique aqueuse contenant un sel de métal des groupes principaux, des métaux de transition ou des lanthanides choisis dans le groupe du lithium, du sodium, du potassium, du magnésium, du calcium, du strontium, de l'aluminium, du titane, du vanadium, du manganèse, du fer, du cobalt, du cuivre, de l'argent, de l'or, du zinc, du cérium, du lanthane, de l'europium, du gadolinium et du dysprosium.

8. Utilisation selon la revendication 7, caractérisée en ce que la préparation cosmétique aqueuse contient un chlorure, un bromure, un sulfate, un acétate, un lactate ou un citrate de lithium, de magnésium, de calcium, d'aluminium, de fer, de cérium ou de lanthane.

9. Préparation d'agent de bronzage de la peau contenant des indolines de formule I selon la revendication 1, ou leurs sels en quantité allant de 0,01 à 10 mmol, de préférence de 0,1 à 3 mmol, et de la dihydroxyacétone en quantité allant de 0,05 à 10 g, de préférence de 0,2 à 5 g, à chaque fois pour 100 g de préparation et un support cosmétique aqueux.
